# EUROPEAN PATENT SPECIFICATION

(11) **EP 4 181 783 B1**
(45) Date of publication and mention of the grant of the patent: **18.03.2026**
(21) Application number: 21734817.6
(22) Date of filing: 21.06.2021
(51) Int. Cl.: A61B 5/145, A61B 5/1477, A61B 5/1491, A61B 5/00

(54) **DEVICE FOR OBTAINING AN INDICATOR OF A MICROCIRCULATORY CONDITION**
VORRICHTUNG ZUM ERHALTEN EINES INDIKATORS FÜR EINEN MIKROZIRKULATIONSZUSTAND
DISPOSITIF D'OBTENTION D'UN INDICATEUR D'UNE MALADIE MICROCIRCULATOIRE

(30) Priority: 14.07.2020 EP 20185601
(43) Date of publication of application: 24.05.2023
(73) Proprietor: Erasmus University Medical Center Rotterdam, 3015 CE Rotterdam (NL)
(72) Inventor: GANGARAM-PANDAY, Norani Hridayanand, 2651 CV Berkel en Rodenrijs (NL); VAN WETERINGEN, Willem, 3011 TT Rotterdam (NL); GOOS, Tomas Gijsbertus, 2628 RN Delft (NL)
(74) Representative: Hepp Wenger Ryffel AG
(86) International application number: PCT/EP2021/066804
(87) International publication number: WO 2022/012872

(56) References cited:
- EP-B1- 2 470 066
- CA-A1- 2 466 105
- CA-C- 2 466 105
- JP-A- S5 460 788
- US-A- 5 355 880
- US-A1- 2002 062 070
- US-A1- 2010 130 842
- US-A1- 2015 099 955
- YITZHAK MENDELSON ET AL: "Noninvasive Transcutaneous Monitoring of Arterial Blood Gases", IEEE TRANSACTIONS ON BIOMEDICAL ENGINEERING, IEEE, USA, vol. BME-19, no. 12, 1 December 1984 (1984-12-01), pages 792 - 800, XP011173582, ISSN: 0018-9294
- VALLEE FABRICE ET AL: "Cutaneous Ear Lobe PCO2 at 37 degrees C To Evaluate Microperfusion in Patients With Septic Shock", BIOSIS, BIOSCIENCES INFORMATION SERVICE, PHILADELPHIA, PA, US, 1 November 2010 (2010-11-01), XP002800689
- DATABASE BIOSIS [online] BIOSCIENCES INFORMATION SERVICE, PHILADELPHIA, PA, US; November 2010 (2010-11-01), VALLEE FABRICE ET AL: "Cutaneous Ear Lobe PCO2 at 37 degrees C To Evaluate Microperfusion in Patients With Septic Shock", Database accession no. PREV201100010891
- CHEST, vol. 138, no. 5, November 2010 (2010-11-01), pages 1062 - 1070, ISSN: 0012-3692(print), DOI: 10.1378/CHEST.09-2690
- DATABASE MEDLINE [online] US NATIONAL LIBRARY OF MEDICINE (NLM), BETHESDA, MD, US; November 1987 (1987-11-01), GOTTRUP F ET AL: "Directly measured tissue oxygen tension and arterial oxygen tension assess tissue perfusion.", Database accession no. NLM3677745
- GOTTRUP F ET AL: "Directly measured tissue oxygen tension and arterial oxygen tension assess tissue perfusion.", CRITICAL CARE MEDICINE NOV 1987, vol. 15, no. 11, November 1987 (1987-11-01), pages 1030 - 1036, ISSN: 0090-3493
- WAXMAN K ET AL: "Transcutaneous oxygen monitoring of emergency department patients", AMERICAN JOURNAL OF SURGERY, PAUL HOEBER, NEW YORK, NY, US, vol. 146, no. 1, 1 July 1983 (1983-07-01), pages 35 - 38, XP026488705, ISSN: 0002-9610, [retrieved on 19830701], DOI: 10.1016/0002-9610(83)90255-6
- ALEXANDRE LIMA ET AL: "Noninvasive monitoring of peripheral perfusion", INTENSIVE CARE MEDICINE, SPRINGER, BERLIN, DE, vol. 31, no. 10, 1 October 2005 (2005-10-01), pages 1316 - 1326, XP019323423, ISSN: 1432-1238, DOI: 10.1007/S00134-005-2790-2
- MARTIN DRES ET AL: "Hemodynamic management of cardiovascular failure by using PCOvenous-arterial difference", JOURNAL OF CLINICAL MONITORING AND COMPUTING, KLUWER ACADEMIC PUBLISHERS, DO, vol. 26, no. 5, 25 July 2012 (2012-07-25), pages 367 - 374, XP035108484, ISSN: 1573-2614, DOI: 10.1007/S10877-012-9381-X
- DATABASE BIOSIS [online] BIOSCIENCES INFORMATION SERVICE, PHILADELPHIA, PA, US; November 2010 (2010-11-01), VALLEE FABRICE ET AL: "Cutaneous Ear Lobe PCO2 at 37 degrees C To Evaluate Microperfusion in Patients With Septic Shock", XP002800689, Database accession no. PREV201100010891
- CHEST, vol. 138, no. 5, November 2010 (2010-11-01), pages 1062 - 1070, ISSN: 0012-3692(print), DOI: 10.1378/CHEST.09-2690
- WERNER BAULIG ET AL: "Clinical Validation of a Digital Transcutaneous PCO2/SpO2 Ear Sensor in Adult Patients after Cardiac Surgery", JOURNAL OF CLINICAL MONITORING AND COMPUTING, KLUWER ACADEMIC PUBLISHERS, DO, vol. 21, no. 5, 16 August 2007 (2007-08-16), pages 303 - 309, XP019529485, ISSN: 1573-2614, DOI: 10.1007/S10877-007-9088-6

## Description

The invention relates to a device for obtaining an indicator of the microcirculatory condition of a patient. Several clinical situations, such as sepsis, haemorrhage and cardiac arrest lead to a decrease in tissue perfusion and affect even the smallest blood vessels. Prolonged microcirculatory impairment eventually leads to dysfunction or even failure of organs, worsening the patient's prognosis.

When bacteria enter the bloodstream and cause a systemic infection to which the body responds, this is called sepsis. Sepsis is one of the main causes of morbidity and mortality in Intensive Care Units (ICU) for neonates, children and adults. The incidence of sepsis is approximately 25% in high-level neonatal ICUs. Multiple factors including prematurity, immunological deficiencies and multiple entry points such as lines/intravascular catheters often lead to infiltration of organisms into the bloodstream.

Clinical monitoring provides continuous surveillance of the patient. Current monitoring techniques are however unable to accurately detect sepsis, in particular the onset. Although there is a large variety in the actual clinical manifestations of sepsis, sepsis is currently defined as a combination of clinical symptoms, deviating lab values and a positive blood culture.

When sepsis is suspected, a blood culture is drawn. Due to the time which is needed for microbial growth and determination in the blood culture, antibiotic treatment is started immediately after withdrawal and the blood culture serves to confirm and assess the specific microbiological profile found in the blood culture and adjust treatment. Unfortunately the clinical condition of the patient may deteriorate severely because of the time delay between blood withdrawal and confirmation of sepsis. Mortality and morbidity are significant despite prompt start of an antibiotic treatment. Early detection of sepsis or even confirming its occurrence remains challenging.

Sepsis is a systemic bloodstream infection by an organism, such as bacteria, causing serious illness. In sepsis, dysfunction of the microcirculation appears in the early stages. Blood flow gets shunted from the arterioles to the venules, leaving the microcirculation hypoxemic and incapable of washing out carbon dioxide. Skin levels of carbon dioxide increase significantly and oxygen levels decrease.

Several studies indicate that sepsis affects the microcirculation at an early stage. Continuous monitoring of the microcirculatory condition would be of added value for detecting potential changes in the microcirculatory condition and relating these changes to sepsis. All cells of the body consume oxygen and produce carbon dioxide. Blood transports oxygen from the lungs to the tissue and stimulates carbon dioxide washout from the tissue which is then exhaled. Due to the lower oxygen and higher carbon dioxide concentrations in peripheral tissues, oxygen diffuses from the circulating blood into the tissue.

Heating of the skin increases blood flow to such an extent that oxygen levels increase and carbon dioxide levels decrease in the heated skin, a process which is called arterialization. When the skin is sufficiently heated and the skin and microcirculatory conditions permit, oxygen and carbon dioxide levels which correspond to arterial oxygen and carbon dioxide levels can be measured on the skin surface. This principle is applied in transcutaneous blood gas monitoring of patients.

There are various techniques for measuring oxygen and carbon dioxide levels transcutaneously. Currently optical and electro-chemical sensors are widely used for transcutaneous oxygen and carbon dioxide measurement.

Typically such sensors house a pH electrode, a reference electrode, an electrolyte solution, a membrane and a heating element. Other technologies for transcutaneous carbon dioxide measurements include optical techniques.

The sensor may be fastened to the skin. The heating element warms the skin to a temperature sufficient to improve perfusion, in particular to a temperature above 40 °C, more particular to a temperature of 42 °C to 45 °C. Oxygen and carbon dioxide diffuse through the epidermis and pass across the sensor's semipermeable membrane into an electrolyte solution-filled sensor chamber.

In the case of an electrochemical sensor, a glass electrode measures the change in pH-value. The electrode's output is converted into a signal indicative of the partial pressure of carbon dioxide. The signal may be read out or displayed.

According to EP2470066 B1 an indicator of tissue perfusion in a patient may be obtained by calculating the difference between the cutaneous partial pressure of carbon dioxide cPCO₂ (here below indicated as PcCO₂) measured with a carbon dioxide sensor without heating of said sensor at a temperature superior to 37.5°C and the arterial pressure of carbon dioxide aPCO₂ below indicated as PaCO₂, or the end-tidal partial pressure of carbon dioxide EtPCO₂ below indicated as PetCO₂

In patients with a ventilation-perfusion mismatch, the end-tidal partial pressure of carbon dioxide might not be an appropriate reference value. In these cases the end-tidal carbon dioxide pressure is not representative of the arterial carbon dioxide pressure.

In specific patient groups such as neonates the small tidal volumes make end-tidal carbon dioxide measurements more difficult and less accurate.

An objective of this invention is to avoid drawbacks of the state of the art and in particular to provide a device and a method for obtaining an indicator of the microcirculatory condition, in particular for indicating and/or predicting sepsis, which in particular allows a noninvasive and reliable measurement providing results within acceptable response times.

According to the invention this object is accomplished by a device according to the independent claims. No method forms part of the claims.

Within this application "microcirculatory condition" means the state of the microcirculatory perfusion and gas diffusion.

The sensor for measuring data indicative of arterial blood carbon dioxide levels may be a sensor for indication of a general or systemic condition.

The sensor for measuring data indicative of tissue carbon dioxide levels may be a sensor for indicating a local condition existing within a patient, e.g. a sensor for measuring data indicative of a skin carbon dioxide level.

The sensors may detect one measured quantity or more measured quantities at a time or one after the other. The measured data may correspond to one or more parameters and/or to a temporal sequence of measured quantities.

A measure of microcirculation provides an indication of the degree of vascularization, blood flow and tissue perfusion and hence of the microcirculatory condition.

The control unit is adapted for determining changes in tissue perfusion based on the measurements of the two sensors.

The control unit is adapted for determining a measure of microcirculation in septic patients, where microcirculation deteriorates due to decreased vascularization and blood flow in the skin and other peripheral tissues.

Transcutaneously measured carbon dioxide levels correspond to arterial carbon dioxide levels when the skin is locally heated. This leads to sufficient arterialization to mitigate influences that would affect skin diffusion, even in the presence of sepsis from which it is known that the diffusion of oxygen and carbon dioxide through the skin is affected by factors that influence the microcirculation.

It has been found that even when tissue perfusion is impaired, arterial blood carbon dioxide levels and heated transcutaneous carbon dioxide levels are in good correspondence; i.e. transcutaneously measured carbon dioxide levels at elevated temperatures correspond to arterial partial pressure of carbon dioxide in arterial blood samples, even in septic neonatal patients.

In contrast, a transcutaneous carbon dioxide measurement that applies no or less heating to the skin is sensitive to changes that directly affect skin diffusion, such as sepsis.

Without sufficient skin heating the transcutaneous carbon dioxide measurement estimates tissue carbon dioxide values in the absence of arterialization. The combination of transcutaneous measurements, in which carbon dioxide is measured on both heated skin and non-heated skin, hence allows measurement and in particular a monitoring of the status of skin carbon dioxide diffusion.

The device detects a relation between the first and the second carbon dioxide level and signals changes thereto to indicate a change of a microcirculatory condition. Specifically, a difference measurement between carbon dioxide levels measured at a first temperature and carbon dioxide levels measured at a second temperature being higher than the first temperature, is used to detect a degree of microcirculatory impairment, caused for example by sepsis.

By independent measuring of a heated transcutaneous carbon dioxide level and tissue carbon dioxide level, it is possible to provide a reliable detection of the microcirculatory condition.

A change of the microcirculatory condition can be a precursor of a severe disturbance of blood flow, vascularization or tissue perfusion. Depending on the temporal course of the change it may therefore indicate a future likelihood of a problem. Hence, preferably, the control unit is adapted for prediction of a measure of microcirculation, preferably adapted for prediction of sepsis depending on the temporal trend of the measured change.

The first sensor and/or the second sensor may comprise a chemical sensor, an opto-chemical sensor, an optical sensor, an electrical sensor, an electro-chemical sensor, an opto-electrical sensor, a pressure sensor and/or a temperature sensor.

The first sensor and the second sensor may in examples outside the scope of the claims be formed by the same sensing unit that works under different conditions, in particular that works intermittently under different conditions.

In this case the sensing unit may for example be used as a sensor for measuring data indicative of tissue blood carbon dioxide levels at a first temperature and as a sensor for measuring data indicative of arterial blood carbon dioxide levels at a second temperature higher than the first temperature.

The sensing unit may be a temperature-controlled sensing unit, in particular for transcutaneous gas measurement, which may be suitable for being used in temperature cycles, such that during a first time period data indicative of first carbon dioxide levels are acquired and during a second time period data indicative of second carbon dioxide levels may be acquired. In this manner temperatures can be cycled alternating.

The sensing unit may also comprise a combined transcutaneous oxygen and carbon dioxide sensor.

According to the invention, the sensor for measuring data indicative of tissue carbon dioxide levels is a first sensing unit and the sensor for measuring data indicative of arterial blood carbon dioxide levels is a second sensing unit different from the first sensing unit. The first sensing unit and/or the second sensing unit may be temperature-controlled. The degree of heating or heatability may be different for the two sensing units.

The first and/or the second sensing unit may comprise a heating element.

Two or more separate sensing units allow a measurement of different data at the same time. Data measured at the same time or at least in a timely manner allow a reasonable comparison of the data.

According to the invention, the two or more sensing units are placed at a distance of at least 1 cm between each other to reduce disturbance of simultaneous measurements.

There are several alternative methods for determining arterial and tissue carbon dioxide levels.

The first and/or the second sensor may be adapted to analyse a blood sample. A blood sample may be taken from the patient. The sample may be drawn from an artery or capillary vessel. For the second sensor the blood can also be drawn from an arterial catheter. Alternatively, a continuous intra-arterial or intravascular measurement may be performed.

Blood samples may also be taken for calibrating a transcutaneous measurement as explained below.

A first sensing unit with a first sensor may comprise a microneedle. The microneedle may be applied to the tissue, preferably up to a distance of 0.2-1 mm from the skin surface. The microneedle may reach the arterioles and/or capillary loops, such that carbon dioxide reaches the needle tip.

The microneedle may be heated or non-heated. The body region around the measurement site is heated or non-heated.

The microneedle may comprise an optical fibre and/or may use fluoroscopy.

The microneedle may comprise an optical fibre and a chemical sensor or an electrochemical probe, for measuring carbon dioxide concentration in a liquid.

The first sensing unit may also be adapted to use spectroscopy for estimation of the tissue carbon dioxide level. The spectroscopy may be proceeded in tissue, hence as an invasive procedure. In a preferred embodiment the second sensor hence comprises a sensor adapted for transcutaneous measurement of carbon dioxide levels, in particular adapted for a heated transcutaneous measurement.

In the context of this application, "heated" measurement means, that the measurement area is heated to a temperature above the normal body temperature, that is to 38°C or above. During a "heated" measurement the measurement area is heated to a temperature which achieves arterialization of the skin to such an extent, such that gas diffusion of carbon dioxide levels are allowed that are measurably closer to blood levels.

The sensing unit comprises a heating element. The second sensing unit may comprise a pH electrode, a reference electrode, an electrolyte solution and a membrane. Preferably the second sensing unit may comprise a heating element.

The first sensor comprises a sensor adapted for transcutaneous measurement of the tissue (or cutaneous) partial pressure of carbon dioxide PcCO₂.

Preferably the device comprises a transcutaneous sensor for measuring data indicative of tissue carbon dioxide levels adapted for non-heated or low-heated transcutaneous measurements of dioxide levels.

For a "non-heated" measurement, the measurement area may also be heated, but not to a temperature that achieves arterialization of the skin, in particular the measurement area may be heated up to 38°C.

The first sensing unit may comprise a pH electrode, a reference electrode, an electrolyte solution and a membrane. Optionally the first sensing unit may comprise a heating element. Stabilizing the temperature of the skin typically provides for reproducible data.

The difference between the cutaneous level of carbon dioxide measured with an unheated transcutaneous sensor, and the cutaneous level of carbon dioxide measured with a heated sensor (as an indicator of the arterial carbon dioxide level) provides information on the carbon dioxide diffusion in the skin. This in turn is a measure of the microcirculatory condition. The microcirculatory condition is impaired in sepsis and can thus be detected with this invention, possibly earlier than current methods.

In particular the device may comprise a sensing unit which is used as a heated transcutaneous sensor and as a non-heated or low-heated transcutaneous sensor.

Hence, the same sensor unit may be used for measuring data indicative of tissue carbon dioxide levels without heating or with limited heating and for measuring data indicative of arterial blood carbon dioxide levels when heated.

Preferably the device comprises at least one further sensor, additional to the first and the second sensor, for measuring data indicative of a further parameter for determining the patient's condition, such as the temperature, the oxygen level, the pH-value of the blood and/or another blood parameter, in particular for correcting and/or calibrating of the transcutaneous blood carbon dioxide level and/or the tissue carbon dioxide level.

The additional sensor may be adapted for measuring data indicative of an arterial blood oxygen level and/or the tissue oxygen level. The additional sensor for indicating an oxygen level may comprise a transcutaneous electrochemical sensor, chemo-optical sensor, an optical sensor or an invasive sensor comprising a needle.

The additional sensor may be adapted for measurement of the temperature, preferably of the skin.

The additional sensor may also be adapted for measuring the foetal haemoglobin, when the device is used for neonates.

The additional sensor may be adapted for measuring the heart rate and/or the heart rate variability.

The additional sensor may be adapted for measuring the pulse rate and/or the pulse rate variability.

The additional sensor may be adapted for measuring the electrical activity, such as ECG values, and/or muscle activity and/or blood flow and/or respiratory gas flow.

The device comprises a heating element.

The first sensor and/or the second sensor and/or if applicable, the additional sensor may be adapted for continuous and/or intermittent and/or alternating measurement.

A continuous measurement allows analysing parameters over time. For parameters known to change slowly or for which a sudden change is not relevant, a continuous measurement may mean that data are collected in predetermined time intervals, such as every few seconds or minutes.

In a preferred embodiment the second sensor and the first sensor are adapted for alternating measurements, preferably with a time lag that ensures the stable presence or absence of arterialization, for example a time lag of at least 10 minutes, to ensure undisturbed measurements, in particular, when measurements are carried out alternating at different temperatures.

In another embodiment of the device the first sensor, and/or the second sensor, and/or if applicable the additional sensor, are arranged in a common housing.

The device may comprise a sensor housing, which may be placed on or in the patient, in particular on the skin. The sensor housing may contain the first sensor, the second sensor and, if applicable, the additional sensors. By placing the sensor housing, all sensors are properly positioned with respect to the patient, in particular all sensors may face the skin.

The sensors may in examples not falling within the scope of the claims be designed as combined sensors, using and/or sharing the same sensor elements, such as a temperature sensor, and/or using and/or sharing the same heating element.

Beneficially the first sensor and the second sensor are adapted to be placed anywhere on the skin or other peripheral tissue, for example on a part of the skin, in particular on an upper or lower extremity, an earlobe, at a fingertip, on a hand palm, on a foot and/or on the thorax.

Advantageously, the control unit is capable of inducing a measurement of the first carbon dioxide level at a first temperature and of inducing a measurement of the second carbon dioxide level, with the measurement area being heated to the second temperature, wherein the second temperature being higher than the first temperature, in particular above 38°C.

Preferably, the control unit is capable of inducing a transcutaneous measurement of the tissue PcCO₂ with a first sensor.

Preferably, the control unit is capable of inducing a transcutaneous measurement of the arterial PaCO₂ with a second sensor.

Preferably, the control unit is capable of controlling the first sensor, the second sensor, and at least one heating element for keeping the measurement site of the skin at a certain temperature.

Preferably, the control unit comprises an output for controlling a heating element, the first sensor and/or the second sensor.

Advantageously the control unit is adapted for estimation of an arterial partial carbon dioxide pressure PaCO₂ based on a transcutaneous partial carbon dioxide pressure, preferably obtained by a measurement by the second sensor, wherein the measurement area is heated above 38°C or to a temperature which causes arterialization of the measurement area.

Advantageously the control unit is adapted for estimation of tissue partial carbon dioxide pressure PcCO₂ based on a transcutaneous partial carbon dioxide pressure, preferably obtained by a measurement by the first sensor, wherein the measurement area is kept at a temperature, for example below 38°C, such that arterialization is not achieved in the measurement area.

In a preferred embodiment of the device the control unit is adapted for determining the difference between the first and the second carbon dioxide level, in particular the difference between the tissue carbon dioxide level and the arterial carbon dioxide level, the ratio of the first and the second carbon dioxide level, in particular the ratio of the tissue carbon dioxide level and the arterial carbon dioxide level and/or an index based on the first and the second carbon dioxide level, for example the tissue carbon dioxide level and the arterial carbon dioxide level, wherein preferably the tissue carbon dioxide levels and/or the arterial carbon dioxide levels are measured transcutaneously.

An index may be determined by a calculation based on the first and second carbon dioxide levels, such as the tissue carbon dioxide levels and the transcutaneous carbon dioxide levels, and optionally further measured or predetermined parameters.

The control unit is adapted for monitoring and in particular for displaying the first and second carbon dioxide levels, such as the tissue carbon dioxide levels and the transcutaneous carbon dioxide levels, the difference, the ratio and/or the index over time and to determine a change over time.

In particular the control unit is adapted for receiving, collecting, storing and processing, particularly time-dependent data.

In particular the control unit is adapted for determining changes in tissue perfusion on a predetermined and/or selectable timescale.

Furthermore the control unit may be adapted for extrapolation of time-dependent data and/or for prediction of for example a tissue perfusion state.

The control unit may be adapted to average and/or to filter measured and/or determined values and/or to determine a running average for a predetermined time interval.

The control unit may be adapted for comparing the first and second carbon dioxide levels, such as the tissue carbon dioxide level and the transcutaneous carbon dioxide level, the difference, the ratio, the index and/or a deduced value with a respective nominal value.

The control unit may be adapted for receiving data by an input device, such as a control panel, a console or a data carrier reader.

In particular the control unit may be adapted for receiving, storing and processing algorithms, correction parameters, nominal values and trigger values.

Correcting parameters may be used to process measured and/or determined data according to a rule. For example medication, vaso-tonic factors and the chemical drift of a sensor may be taken into account for analysing measured data.

The control unit may be adapted for indicating a disturbance of a sensor, a need for maintenance or a need for a new sensor calibration.

If the difference of a measured and/or determined value and a respective nominal value exceeds a predetermined trigger value, the control unit may produce a respective output information.

The control unit may analyse time courses of tissue carbon dioxide levels, transcutaneous carbon dioxide levels, differences, ratios, indexes and/or deduced values and may detect a critical condition or a likelihood of a critical condition in the microcirculatory condition.

The control unit may provide an alert signal, if there is a critical condition in the microcirculatory or a likelihood of a critical condition to be expected.

The device may provide a result based on a present measured and/or determined value. The device may provide a result based on a time development of a measured and/or determined value.

The control unit may be adapted to determine a difference, a ratio and/or an index on the basis of data measured during a time interval. Preferably the control unit may be adapted for a continuous determination on the basis of stored data of a running time interval. For this purpose the device may comprise a storage device for storing measurement data and/or reference data.

Thus the device allows for a retrospective analysis of the data.

Changes due to sepsis are caused by slow processes. Thus, the result to be determined will take some time, typically a few minutes to a few hours. However, the device may provide an indicator of the microcirculatory condition and thereby an indicator of sepsis, potentially earlier than clinical symptoms indicate or the clinical manifestation of sepsis.

In a preferential embodiment of the device the control unit is connected or connectable to an output device, such as a monitor or a display, preferably for displaying the measure of microcirculation and/or for displaying signals produced by the control unit.

The output device is a part of the device.

The output device may be adapted to emit an acoustic and/or optical signal.

The output device may be adapted to display measured and/or determined values and/or a time-dependent representation of measured and/or determined values.

The output device is adapted to indicate a measure of microcirculation. For example the output device may comprise a display being designed to comprise a three-level scale, wherein the first level indicates "no problem", the second level indicates "likelihood of a problem" and the third level indicates "attention: problem".

The output device may be adapted to display a measure of the quality of measured and/or determined values, such as an estimated measurement error or a standard deviation of an averaged value.

The output device may be adapted to display a measure of the quality of the measure of the microcirculation, such as a confidence index, which may be based on the quantity of data used.

The more data used, the more trustworthy the result will be.

Such a confidence index may be derived from a deviation of measurement data over a period of time; i.e. if the deviation is relatively low over a sustained period of time, the confidence index will be high.

The values measured by the described sensors may be displayed by the output devices. Thus, the user gets additional information and he can control the quality of the measure of microcirculation.

The device may be a stand-alone device to be placed at the bedside of a patient. The device may also be a hook-up element for an existing system.

The device may be partly formed by components of an existing system and may for example share sensors or an output device with an existing system.

The object of the disclosure is also accomplished by a method not falling within the scope of the claims for obtaining an indicator of tissue perfusion, comprising the following steps.

A first carbon dioxide level, in particular a tissue carbon dioxide level, of a patient is provided, preferably a transcutaneously measured tissue PcCO₂ value.

A second carbon dioxide level, in particular an arterial blood carbon dioxide level, of a patient is provided, preferably a transcutaneously measured arterial carbon dioxide value tcPCO₂.

Preferably the first and second carbon dioxide levels, such as the tissue carbon dioxide levels and the arterial blood carbon dioxide levels, are provided by independent measurements.

A measure for microcirculation is determined, preferably in septic patients, on the basis of the first and the second carbon dioxide levels, such as the tissue carbon dioxide level and the arterial blood carbon dioxide level.

Preferably the measure for microcirculation is displayed in real-time.

The first and the second carbon dioxide levels may be provided by a data collection or may be measured, preferably by transcutaneous measurements. Other measurement principles may be used as explained above.

Preferably the first carbon dioxide level is measured at a first temperature and the second carbon dioxide level is measured with the measurement area being heated to a second temperature higher than the first temperature, preferably above 38°.

Preferably the second carbon dioxide level, in particular the arterial blood carbon dioxide level, is measured by transcutaneously measuring the arterial PaCO₂ with the measurement area being heated to a temperature above 38°C, preferably above 40°C, such that arterialization of the skin is achieved, preferably heated by a heating element of a sensing unit and/or arranged in a sensor head.

Preferably the first carbon dioxide level, in particular the tissue carbon dioxide level, is measured by transcutaneously measuring the tissue PcCO₂. Preferably the measurement area is kept at the temperature of the patient, for example below 38°C and/or at 37.5°C. The measurement area is kept at the temperature which does not causes arterialization of the skin. When heat is applied metabolic activity changes and arterialization starts.

The measurements can be performed at the same time or alternating, preferably with a time lag that ensures the stable presence or absence of arterialization, for example with a time lag of at least 10 minutes.

The object of the disclosure is also accomplished by a computer program product not falling within the scope of the claims directly loadable into the internal memory of a digital comprising software code portions for performing the steps of a method for obtaining an indicator of tissue perfusion as described above, when said product is run on a computer.

The computer program may be loaded and/or run on a control unit of a device as described above.

The computer program may be loaded and/or run on a central computer device or may be loaded and/or run on a measurement device for measuring an arterial blood carbon dioxide level and/or a tissue carbon dioxide level of the patient.

The invention is further explained with reference to preferred embodiments and the following drawings which show:
- Fig. 1:: a schematic representation of a first example of a device;
- Fig. 2:: a schematic representation of a second example of a device;
- Fig. 3:: a schematic view on a sensor head of a third example for a device;
- Fig. 4:: a schematic cross-sectional view of the sensor head of figure 3;
- Fig. 5:: a schematic representation of a fourth example of a device;
- Fig. 6:: a schematic cross-sectional view of the sensor head of the fourth example of a device;
- Fig. 7:: a schematic representation of an example of microcirculatory conditions as measured by a device.

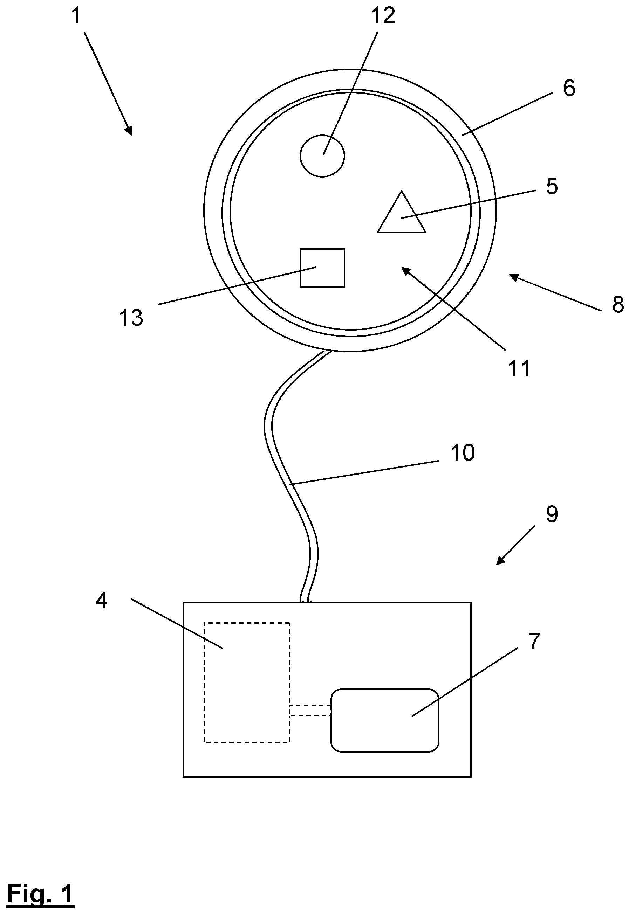

Figure 1 shows a schematic representation of a first example of a device 1 for obtaining an indicator of microcirculatory condition of a patient. The device 1 comprises a first sensor 13 measuring data indicative of tissue carbon dioxide levels and a second sensor 12, for transcutaneously measuring data indicative of arterial blood carbon dioxide levels.

The second sensor 12 is formed by a second sensing unit and the first sensor 13 is formed by a first sensing unit different from the second sensing unit.

The second sensor 12 and the first sensor 13 are arranged in a common housing 6 forming a sensor head 8.

The device 1 comprises a control unit 4 for determining a measure of microcirculation, in particular changes in tissue perfusion or microcirculatory condition, on the basis of values, measured by the first sensor 13 and the second sensor 12, in particular on the basis of the tissue carbon dioxide level and the heated transcutaneously measured carbon dioxide level.

The device 1 comprises an additional sensor 5 for measuring data indicative of a further parameter, such as the temperature, the oxygen level, the pH-value of the blood and/or another blood parameter, in particular for correcting the arterial blood carbon dioxide level and/or the tissue carbon dioxide level.

The sensor head 8 may be connected to a device base 9 by a cable 10. The control unit 4 may be arranged in the device base 9.

The control unit 4 is connected to an output device 7, such as a monitor or a display for displaying the measure of microcirculation. The output device 7 may also be arranged in the device base 9.

The sensor head 8 comprises a contact face 11 which is directa-ble towards a measuring site. In this case, the measuring site is an area on the skin of a patient.

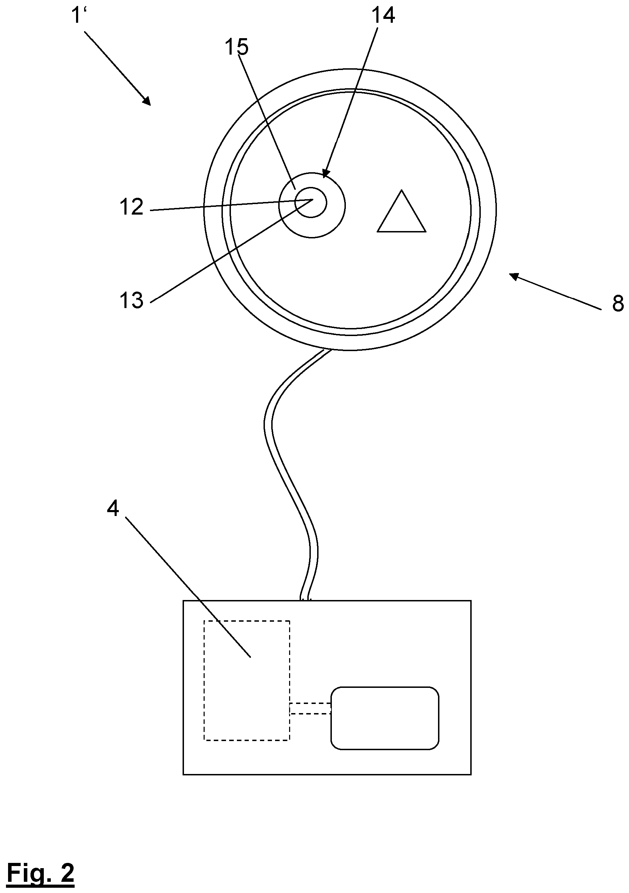

Figure 2 shows a schematic representation of a second example of a device 1' for obtaining an indicator of microcirculatory condition of a patient.

The device 1' comprises a sensor head 8 with a single sensing unit 14, comprising first and second sensors 13 and 12 respectively. Depending on the temperature, the sensing unit 14 is controlled for transcutaneously measuring data indicative of arterial blood carbon dioxide levels or indicative of tissue carbon dioxide levels. Skin temperature is controlled by a heating element 15 controlled by the control unit 4. Hence, the sensing unit 14 acts as a sensor for measuring data indicative of arterial blood carbon dioxide levels at a first temperature and as a sensor for measuring data indicative of tissue carbon dioxide levels at a second temperature.

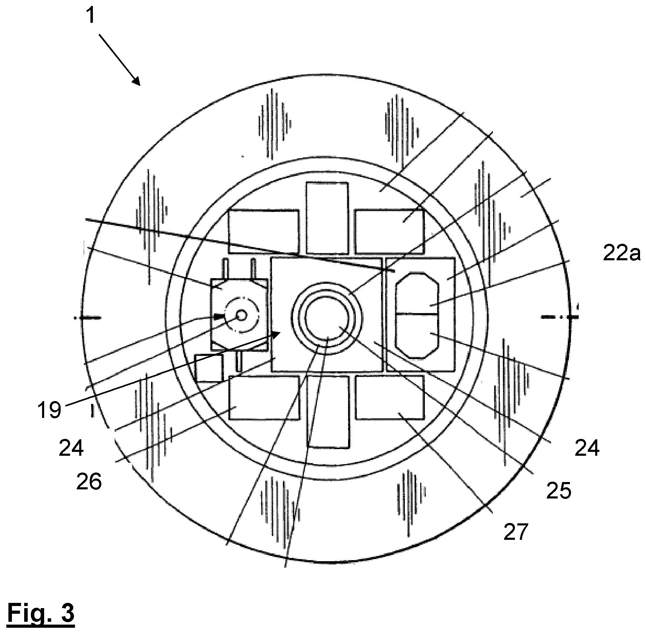

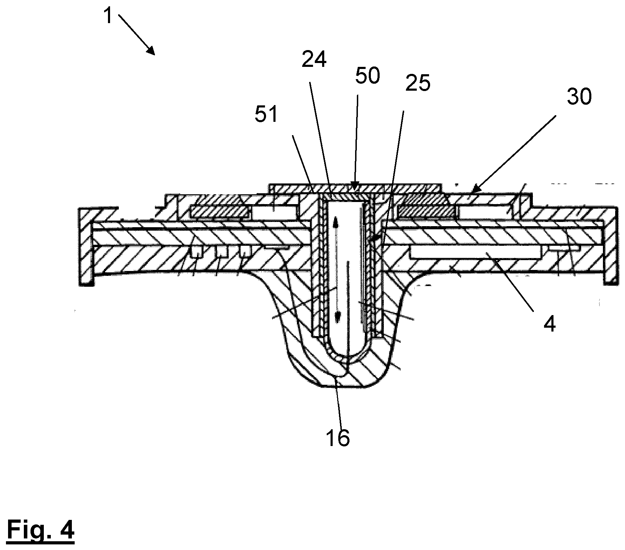

The device 1 shown in FIGS. 3 and 4 comprises a surface 30 over which, in the embodiment shown, a membrane 50 is arranged and there between a thin layer of electrolyte 51. This membrane 50 is placed on the skin at a point of the human body which has a good blood flow, for example at a finger, at the forehead or at the earlobe (see figure 4).

The device 1 shown includes a sensor unit 19 adapted for transcutaneous measurement of the first and second tissue carbon dioxide level by electrochemical detection. The device 1 to this end preferably includes a micro-pH electrode 24 as well as an Ag/AgCl reference electrode 25 (see Fig 3). The carbon dioxide level can be measured potentiometrically in that the pH of the thin layer of the electrolyte solution 51 is measured which is in communication with the skin via the hydrophobic membrane 50 which has good gas permeability. A change in the carbon dioxide value at the skin surface effects a pH change of the electrolyte solution. The pH is measured in that the potential is measured between the miniature pH electrode 24 and a reference electrode 25. The micro-pH electrode 24 is conductively connected via the electrical inner deflector 16 to the control unit 4.

The device 1 comprises a heating element 26 and a temperature sensor 27.

The sensor unit 19 is adapted for a transcutaneous measurement at a first temperature, wherein the measurement area is kept below 38°C.

The sensor unit 19 is also adapted for a transcutaneous measurement at a second temperature being higher than the first temperature, wherein the measurement area is heated above 38°C by the heating element 26.

Thus the sensor unit 19 is a first sensor for measuring data indicative of first carbon dioxide levels, in particular tissue carbon dioxide levels, and a second sensor for measuring data indicative of second carbon dioxide levels, in particular arterial blood carbon dioxide levels.

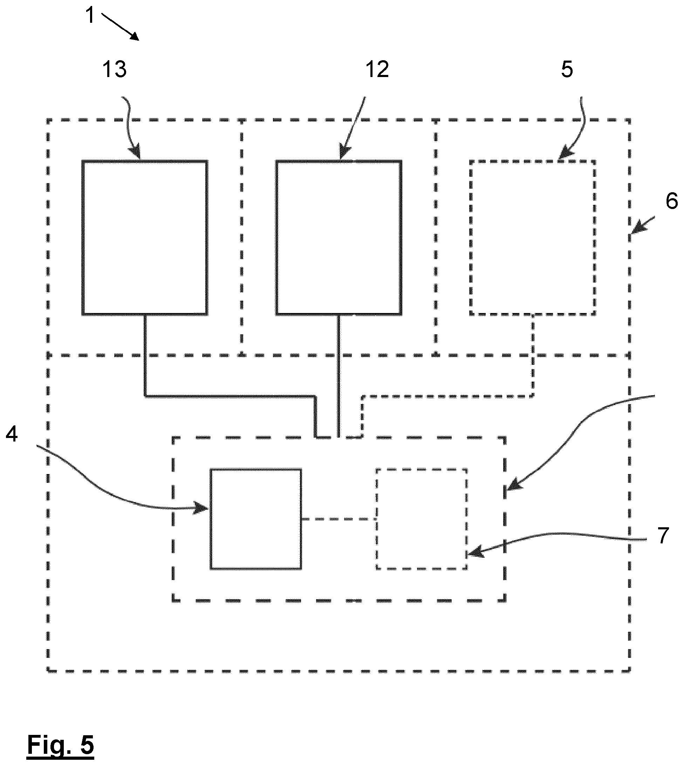

Figure 5 shows a schematic representation of a fourth example of a device 1 for obtaining an indicator of microcirculatory condition of a patient.

A first sensing unit 13, a second sensing unit 12, an additional sensor 5, a housing of a processor or control unit 4 and an output device 7 may be arranged in a common housing 6.

Preferably the housing allows a combination, integration and suitable separation of components.

The first sensing unit 13 may be a first transcutaneous measurement device. The second sensing unit 12 may be a second transcutaneous measurement device and may comprise a heating element

The additional sensor 5 may detect temperature, transcutaneous O₂ and/or may comprise an input for external values.

The output device 7 may comprise a display for showing a digital or analogue output.

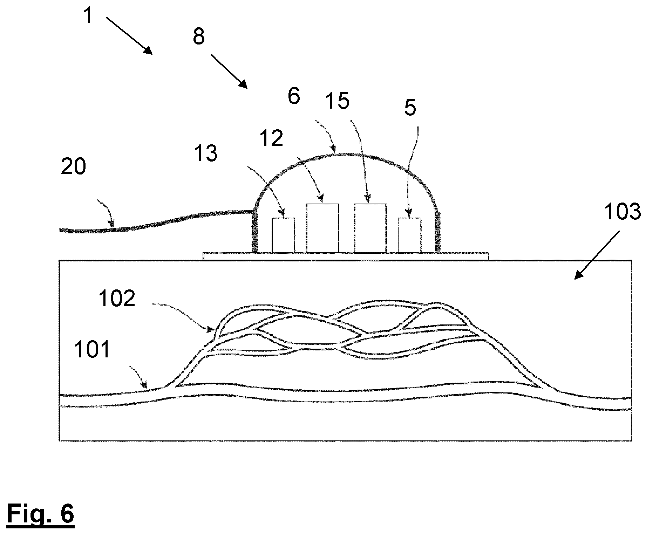

Figure 6 shows a schematic cross-sectional view of a sensor head 8 for obtaining an indicator of a microcirculatory condition of a patient in a fourth example of a device 1.

The sensor head 8 is placed in contact to the skin 103 of a patient.

A first sensing unit 13 for measuring data indicative of a tissue carbon dioxide level, an additional sensor 5, a second sensing unit 12 for measuring data indicative of an arterial carbon dioxide level and a heating element 15 are arranged in a common housing 6. The sensing unit 13 is adapted for a transcutaneous carbon dioxide measurement at a first temperature, wherein the measurement area is kept below 38°C. The sensing unit 12 is adapted for a transcutaneous carbon dioxide measurement at a second temperature being higher than the first temperature, wherein the measurement area is heated above 38°C. A processor or control unit 4 and an output device 7 (see figure 4), not shown in this figure, may also be arranged in the housing 6. The processor 4 is programmed to detect changes in skin perfusion and has an input for receiving a measured or estimated first carbon dioxide level value, in particular a tissue carbon dioxide level value from the first sensor 13, an input for receiving a measured or estimated second carbon dioxide level value from the second sensor 12, in particular an arterial blood carbon dioxide level value; and at least one output interface for submitting an indicator of a microcirculatory condition of a patient based on the received inputs.

The sensor head 8 is connected to an output connection 20, which may establish a connection to an external processor or controller and which may act as a power supply.

The skin 103 is permeated with arteries 101 and capillaries 102.

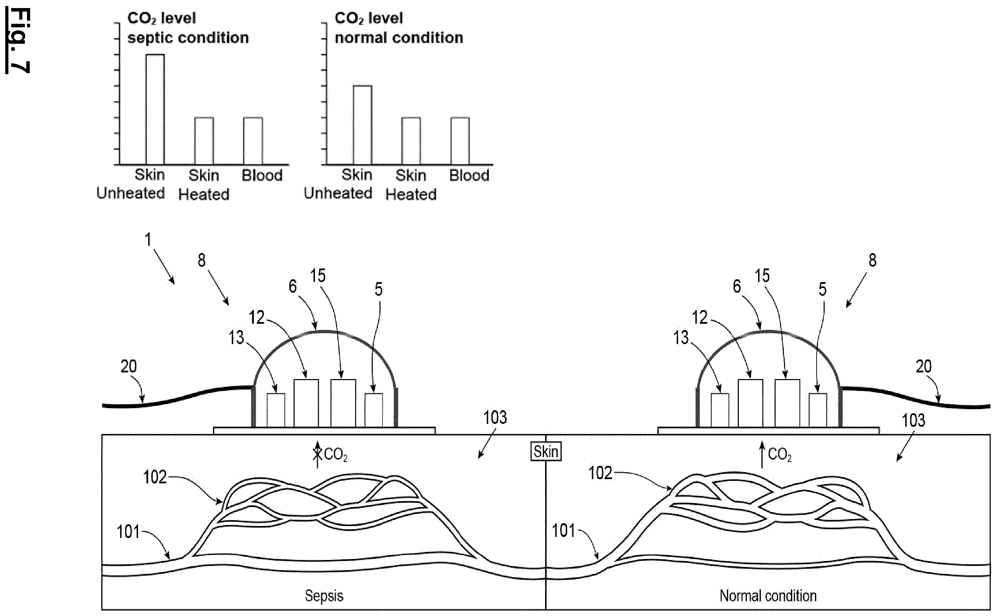

Figure 7 shows a schematic representation of an example of microcirculatory conditions as measured by a first sensor 13 and a second sensor 12 under condition of sepsis and under normal condition.

Blood supply in the skin arteries 101 remains unimpaired during sepsis.

However, blood supply in the capillaries 102 may be impaired in a septic condition. Consequently a significantly different amount of carbon dioxide diffuses from the skin surface, which may be detected by a transcutaneous measurement without heating the skin.

Surprisingly, heating of the skin leads to sufficient arterialization to mitigate influences that would affect skin diffusion, even in the presence of sepsis. Thus, carbon dioxide levels measured transcutaneously while heating the skin correlate with arterial carbon dioxide levels.

A septic condition indicator can be outputted by the processor e.g. by outputting the difference between the first and the second carbon dioxide level, for example the difference between tissue carbon dioxide level and the arterial carbon dioxide oxygen level, the ratio of the first and the second carbon dioxide level, for example the ratio of the tissue carbon dioxide level and the arterial blood carbon dioxide level and/or an index based on the first and the second carbon dioxide level, for example the tissue carbon dioxide level and the arterial blood carbon dioxide level, preferably for a prediction of sepsis.

## Claims

1. Device (1) for obtaining an indicator of a microcirculatory condition of a patient, comprising
- a heating element (15),
- at least one first sensor (13) for measuring data indicative of tissue carbon dioxide levels, the first sensor (13) comprising a measurement area of the first sensor to be contacted with the patient's tissue, and adapted for a transcutaneous carbon dioxide measurement at a first temperature, wherein the measurement area is kept below 38°C,
- at least one second sensor (12) for measuring data indicative of arterial blood carbon dioxide levels, the second sensor (12) comprising a measurement area of the second sensor to be contacted with the patient's tissue and adapted for a transcutaneous carbon dioxide measurement at a second temperature being higher than the first temperature, wherein heating element is adapted to heat the measurement area of the second sensor above 38°C such that arterialization of the skin is achieved, and
- a control unit (4) for detecting changes in skin perfusion, the control unit (4) having at least one input for receiving a measured or estimated tissue carbon dioxide level value from the first sensor (13), at least one input for receiving a measured or estimated arterial blood carbon dioxide level value from the second sensor (12); and at least one output interface for outputting an indicator of a microcirculatory condition of a patient based on the received inputs,
wherein the control unit is adapted for determining the indicator of a microcirculatory condition of a patient based on
- the difference between the tissue carbon dioxide level and the arterial blood carbon dioxide level,
- the ratio between the tissue carbon dioxide level and the arterial blood carbon dioxide level
and/or
- an index based on the tissue carbon dioxide level and the arterial blood carbon dioxide level,
and wherein the first sensor is a first sensing unit (13) and the second sensor is a second sensing unit (12) different from the first sensing unit (13),
wherein the control unit is adapted for monitoring and displaying the tissue carbon dioxide levels and the arterial blood carbon dioxide levels, the difference, the ratio and/or the index over time and to determine a change over time,
wherein the output interface of the control unit (4) is connected or connectable to an output device, such as a monitor or a display
wherein the control unit is adapted for prediction of sepsis depending on the temporal trend of a measured change of the microcirculatory condition and
wherein the sensing units are placed at a distance of at least 1 cm between each other and arranged in a common housing (6).

2. Device according to claim 1, wherein the device (1, 1') comprises at least one additional sensor (5) adapted for measuring data indicative of a further parameter, such as a temperature, the oxygen level or a pH-value of the blood and/or another blood parameter, in particular for correcting arterial blood carbon dioxide levels, in particular the arterial blood carbon dioxide levels, and/or the tissue carbon dioxide levels.

3. Device according to one of the preceding claims, wherein the sensors (12, 13, 5) are adapted for continuous and/or intermittent and/or alternating measurement.

4. Device according to one of the preceding claims, wherein the first sensor (13) and the second sensor (12) are adapted to be placed on a part of the skin, in particular on an earlobe, at a fingertip, on a hand palm, on a foot and/or on the thorax.

5. Device according to one of the preceding claims, wherein the control unit (4) is adapted for inducing the measurement of the tissue carbon dioxide level value at the first temperature and for inducing the measurement of the arterial blood carbon dioxide level at the second temperature wherein the control unit is adapted for controlling the heating element for keeping the measurement site of the skin at a certain temperature and wherein
the control unit comprises an output for controlling the heating element, the first sensor and/or the second sensor.

6. Device according to one of the preceding claims, wherein the control unit (4) is adapted for estimation of an arterial partial carbon dioxide pressure PaCO₂ based on a transcutaneous partial carbon dioxide pressure tcPCO₂, obtained by a measurement by the second sensor (12)

7. Device according to one of the preceding claims, wherein the control unit (4) is adapted for estimation of tissue partial carbon dioxide pressure PcCO₂ based on a transcutaneous partial carbon dioxide pressure P_{C}CO₂, obtained by a measurement by the first sensor (13).
vel and/or a temperature.

## Patentansprüche

1. Vorrichtung (1) zum Erhalten eines Indikators für einen mikrozirkulatorischen Zustand eines Patienten, umfassend ein Heizelement (15),
mindestens einen ersten Sensor (13) zum Messen von Daten, die für Gewebe-Kohlendioxidspiegel indikativ sind, wobei der erste Sensor (13) einen Messbereich des ersten Sensors umfasst, der mit dem Gewebe des Patienten in Kontakt zu bringen ist, und für eine transkutane Kohlendioxidmessung bei einer ersten Temperatur ausgelegt ist, wobei der Messbereich unter 38 °C gehalten wird,
mindestens einen zweiten Sensor (12) zum Messen von Daten, die für arterielle Blut-Kohlendioxidspiegel indikativ sind, wobei der zweite Sensor (12) einen Messbereich des zweiten Sensors umfasst, der mit dem Gewebe des Patienten in Kontakt zu bringen ist, und für eine transkutane Kohlendioxidmessung bei einer zweiten Temperatur ausgelegt ist, die höher als die erste Temperatur ist, wobei das Heizelement dafür ausgelegt ist, den Messbereich des zweiten Sensors auf über 38 °C zu erwärmen, so dass eine Arterialisierung der Haut erreicht wird, und
eine Steuereinheit (4) zum Erfassen von Änderungen der Hautperfusion, wobei die Steuereinheit (4) mindestens einen Eingang zum Empfangen eines gemessenen oder geschätzten Werts des Gewebe-Kohlendioxidspiegels vom ersten Sensor (13), mindestens einen Eingang zum Empfangen eines gemessenen oder geschätzten Werts des arteriellen Blut-Kohlendioxidspiegels vom zweiten Sensor (12); und mindestens eine Ausgangsschnittstelle zum Ausgeben eines Indikators für einen mikrozirkulatorischen Zustand eines Patienten basierend auf den empfangenen Eingaben aufweist,
wobei die Steuereinheit dafür ausgelegt ist, den Indikator für einen mikrozirkulatorischen Zustand eines Patienten zu bestimmen, basierend auf
o der Differenz zwischen dem Gewebe-Kohlendioxidspiegel und dem arteriellen Blut-Kohlendioxidspiegel,
o dem Verhältnis zwischen dem Gewebe-Kohlendioxidspiegel und dem arteriellen Blut-Kohlendioxidspiegel
und/oder
einem Index, der auf dem Gewebe-Kohlendioxidspiegel und dem arteriellen Blut-Kohlendioxidspiegel basiert,
und wobei der erste Sensor eine erste Sensoreinheit (13) ist und der zweite Sensor eine zweite Sensoreinheit (12) ist, die sich von der ersten Sensoreinheit (13) unterscheidet,
wobei die Steuereinheit dafür ausgelegt ist, die Gewebe-Kohlendioxidspiegel und die arteriellen Blut-Kohlendioxidspiegel, die Differenz, das Verhältnis und/oder den Index über die Zeit zu überwachen und anzuzeigen und eine Änderung über die Zeit zu bestimmen,
wobei die Ausgangsschnittstelle der Steuereinheit (4) mit einer Ausgabevorrichtung, wie einem Monitor oder einer Anzeige, verbunden oder verbindbar ist,
wobei die Steuereinheit für die Vorhersage von Sepsis in Abhängigkeit vom zeitlichen Trend einer gemessenen Änderung des mikrozirkulatorischen Zustands ausgelegt ist und
wobei die Sensoreinheiten in einem Abstand von mindestens 1 cm zueinander platziert und in einem gemeinsamen Gehäuse (6) angeordnet sind.

2. Vorrichtung nach Anspruch 1, wobei die Vorrichtung (1, 1') mindestens einen zusätzlichen Sensor (5) umfasst, der zum Messen von Daten ausgelegt ist, die für einen weiteren Parameter indikativ sind, wie eine Temperatur, den Sauerstoffspiegel oder einen pH-Wert des Blutes und/oder einen anderen Blutparameter, insbesondere zur Korrektur von arteriellen Blut-Kohlendioxidspiegeln, insbesondere der arteriellen Blut-Kohlendioxidspiegel, und/oder der Gewebe-Kohlendioxidspiegel.

3. Vorrichtung nach einem der vorhergehenden Ansprüche, wobei die Sensoren (12, 13, 5) für eine kontinuierliche und/oder intermittierende und/oder alternierende Messung ausgelegt sind.

4. Vorrichtung nach einem der vorhergehenden Ansprüche, wobei der erste Sensor (13) und der zweite Sensor (12) dafür ausgelegt sind, an einem Teil der Haut platziert zu werden, insbesondere an einem Ohrläppchen, an einer Fingerspitze, auf einer Handfläche, an einem Fuss und/oder am Thorax.

5. Vorrichtung nach einem der vorhergehenden Ansprüche, wobei die Steuereinheit (4) dafür ausgelegt ist, die Messung des Werts des Gewebe-Kohlendioxidspiegels bei der ersten Temperatur zu veranlassen und die Messung des arteriellen Blut-Kohlendioxidspiegels bei der zweiten Temperatur zu veranlassen,
wobei die Steuereinheit dafür ausgelegt ist, das Heizelement zu steuern, um die Messstelle der Haut auf einer bestimmten Temperatur zu halten, und wobei
die Steuereinheit einen Ausgang zur Steuerung des Heizelements, des ersten Sensors und/oder des zweiten Sensors umfasst.

6. Vorrichtung nach einem der vorhergehenden Ansprüche, wobei die Steuereinheit (4) zur Kalkulation eines arteriellen Kohlendioxid-Partialdrucks PaCO2 basierend auf einem transkutanen Kohlendioxid-Partialdruck tcPCO2, der durch eine Messung durch den zweiten Sensor (12) erhalten wird, ausgelegt ist.

7. Vorrichtung nach einem der vorhergehenden Ansprüche, wobei die Steuereinheit (4) zur Kalkulation eines Gewebe-Kohlendioxid-Partialdrucks PcCO2 basierend auf einem transkutanen Kohlendioxid-Partialdruck PcCO2, der durch eine Messung durch den ersten Sensor (13) erhalten wird, ausgelegt ist.

## Revendications

1. Dispositif (1) pour obtenir un indicateur d'un état microcirculatoire d'un patient, comprenant
un élément chauffant (15),
au moins un premier capteur (13) destiné à mesurer des données indicatives de niveaux de dioxyde de carbone tissulaire, le premier capteur (13) comprenant une zone de mesure du premier capteur destinée à être mise en contact avec le tissu du patient, et adapté pour une mesure transcutanée de dioxyde de carbone à une première température, dans lequel la zone de mesure est maintenue en dessous de 38 °C,
au moins un second capteur (12) destiné à mesurer des données indicatives de niveaux de dioxyde de carbone du sang artériel, le second capteur (12) comprenant une zone de mesure du second capteur destinée à être mise en contact avec le tissu du patient et adapté pour une mesure transcutanée de dioxyde de carbone à une seconde température, ladite seconde température étant supérieure à la première température, dans lequel l'élément chauffant est adapté pour chauffer la zone de mesure du second capteur au-dessus de 38 °C de manière à obtenir une artérialisation de la peau, et
une unité de commande (4) adapté à détecter des variations de la perfusion cutanée, l'unité de commande (4) comprenant au moins une entrée destinée à recevoir une valeur mesurée ou estimée du niveau de dioxyde de carbone tissulaire provenant du premier capteur (13), au moins une entrée pour recevoir une valeur mesurée ou estimée du niveau de dioxyde de carbone du sang artériel provenant du second capteur (12) ; et au moins une interface de sortie destiné à fournir un indicateur d'un état microcirculatoire du patient sur la base des entrées reçues,
dans lequel l'unité de commande est configurée pour déterminer l'indicateur d'un état microcirculatoire du patient sur la base de :
o la différence entre le niveau de dioxyde de carbone tissulaire et le niveau de dioxyde de carbone du sang artériel,
o le rapport entre le niveau de dioxyde de carbone tissulaire et le niveau de dioxyde de carbone du sang artériel,
et/ou
un indice basé sur le niveau de dioxyde de carbone tissulaire et le niveau de dioxyde de carbone du sang artériel,
et dans lequel le premier capteur est une première unité de détection (13) et le second capteur est une seconde unité de détection (12), ladite seconde unité de détection (12) étant différente de la première unité de détection (13),
dans lequel l'unité de commande est adaptée pour surveiller et afficher les niveaux de dioxyde de carbone tissulaire et les niveaux de dioxyde de carbone du sang artériel, la différence, le rapport et/ou l'indice en fonction du temps, et pour déterminer une variation au cours du temps,
dans lequel l'interface de sortie de l'unité de commande (4) est connectée ou peut être connectée à un dispositif de sortie, tel qu'un moniteur ou un écran,
dans lequel l'unité de commande est adaptée pour la prédiction d'une septicémie en fonction de l'évolution temporelle d'une variation mesurée de l'état microcirculatoire et
dans lequel les unités de détection sont disposée à une distance d'au moins 1 cm l'une de l'autre et agencées dans un boîtier commun (6).

2. Dispositif selon la revendication 1, dans lequel le dispositif (1, 1') comprend au moins un capteur supplémentaire (5) configuré pour mesurer des données indicatives d'un paramètre supplémentaire, tel qu'une température, un niveau d'oxygène ou une valeur de pH du sang et/ou un autre paramètre sanguin, notamment pour corriger les niveaux de dioxyde de carbone du sang artériel, en particulier les niveaux de dioxyde de carbone du sang artériel, et/ou les niveaux de dioxyde de carbone tissulaire.

3. Dispositif selon l'une quelconques des revendications précédentes, dans lequel les capteurs (12, 13, 5) sont adaptés pour une mesure continue et/ou intermittente et/ou alternée.

4. Dispositif selon l'une quelconques des revendications précédentes, dans lequel le premier capteur (13) et le second capteur (12) sont configurés pour être placés sur une partie de la peau, en particulier sur un lobe d'oreille, à l'extrémité d'un doigt, sur la paume de la main, sur un pied et/ou sur le thorax.

5. Dispositif selon l'une quelconques des revendications précédentes, dans lequel l'unité de commande (4) est configurée pour déclencher la mesure de la valeur du niveau de dioxyde de carbone tissulaire à la première température et pour déclencher la mesure du niveau de dioxyde de carbone du sang artériel à la seconde température,
dans lequel l'unité de commande est adaptée pour commander l'élément chauffant afin de maintenir le site de mesure de la peau à une certaine température et dans lequel l'unité de commande comprend une sortie configurée pour commander l'élément chauffant, le premier capteur et/ou le second capteur.

6. Dispositif selon l'une quelconques des revendications précédentes, dans lequel l'unité de commande (4) est configurée pour estimer une pression partielle artérielle en dioxyde de carbone PaCO2 sur la base d'une pression partielle transcutanée en dioxyde de carbone tcPCO2, obtenue par une mesure effectuée par le second capteur (12).

7. Dispositif selon l'une quelconques des revendications précédentes, dans lequel l'unité de commande (4) est configurée pour estimer une pression partielle tissulaire en dioxyde de carbone PcCO2 sur la base d'une pression partielle transcutanée en dioxyde de carbone PcCO2, obtenue par une mesure par le premier capteur (13).
